(19) Europäisches Patentamt
European Patent Office
Office européen des brevets

(11) **EP 2 020 250 A1**

(12) **EUROPEAN PATENT APPLICATION**

(43) Date of publication:
**04.02.2009 Bulletin 2009/06**

(51) Int Cl.:
**A61N 2/00** *(2006.01)*

(21) Application number: **07113241.9**

(22) Date of filing: **26.07.2007**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(71) Applicant: **Cuppen, Johannes Josephus Maria
5508 HB Veldhoven (NL)**

(72) Inventor: **Cuppen, Johannes Josephus Maria
5508 HB Veldhoven (NL)**

(74) Representative: **van Looijengoed, Ferry Antoin
Theodorus et al
De Vries & Metman
Overschiestraat 180
1062 XK Amsterdam (NL)**

(54) **Device and method for electromagnetic stimulation of a process within living organisms**

(57) Device for applying an electromagnetic field for stimulation of a process within a living organism when applied to at least part of a body, comprising a driving means for generating a time varying drive signal and a transducer pre-sponsive to said drive signal for generating a time varying electromagnetic field signal B(t). The signal B(t) comprises one periodic base signal $b_i(t)$ or a superposition of two or more periodic base signals $b_i(t)$ (i=1,2,3,...). The signal $b_i(t)$ is defined as: $b_i(t)=a_1*(2\exp(-\omega_i t)-(1+\exp(-\omega_i T_i/2))$ for $0 \leq t \leq T_i/2$ and $b_i(t)=-b_i(t-T_i/2)$ for $T_i/2 \leq t \leq T_i$, wherein $T_i$ is the period of $b_i(t)$, $a_i$ is an amplitude of $b_i(t)$ and $\omega_i$ is a characteristic frequency determining the shape of the signal $b_i(t)$.

Fig. 2

EP 2 020 250 A1

## Description

Field of the invention

The invention relates to stimulating a process within living organisms using electromagnetic fields. In particular, the invention relates to a device which is suitable for effective stimulation of processes, in particular the immune response, in living organisms.

Background of the invention

It is well known that time varying low-energy electric or magnetic fields produce a range of responses in biological systems. Based on these responses various therapeutic or bio-stimulation treatments using low frequency and low-energy electromagnetic fields have been proposed. For example, US 3,890,953 describes a method for the stimulation of bone growth and other tissues. In US 5,183,456 a method for regulation of the growth of cancer cells is described. In US 5,290,409 a method is described in which the transport of several types of ions can be influenced.

An interesting group of studies have demonstrated that human and murine macrophages can be stimulated to higher activity through low frequency electromagnetic field exposure (see Simkó et al., Eur. J. Cell Biol. Vol. 80, 2001, p. 562-566 and Lupke et al., Free Radic. Res. Vol. 38, 2004, p. 985-993). Several authors have demonstrated that the observed production of cytokines, increased immune parameters and stress effects were initiated by exposure to electromagnetic fields. From these studies it was concluded that low field electromagnetic field exposure causes stress at the cellular level, leading to production of cytokines and consequently biological response, including immune response (see Blank et al., Bio-electrochem. and Bioenerg., Vol. 33, p. 109-114, 1992 and Mol. Biol. Cell Vol. 6, p. 466a, 1995; Goodman et al., Bioelectro-chem. and Bioenerg., Vol. 33 p. 115; Simkó et al., J. Cell. Biochem. Vol. 93, 2004, p. 83-92; Monselise et al., Biochem. & Biophys. Res. Com. Vol. 302(2), p. 427-434, 2004; De Bruyn et al., Environ. Res., Vol. 65 (1) p. 149-160, 1994; Markov et al. in Bioelectromagnetics edited by S.N. Ayrapetryan and M.S. Markov (eds.), Springer 2006, p. 213-225).

Proper stimulation of the immune response leads to improved resistance against infectious diseases and thus positively affects the health of the exposed organism. This insight opens new possibilities for (preventive) treatment in large, dense populations wherein infectious diseases are an increasing problem. Such problems are especially prevalent in populations with genetically uniform organisms such as farmed livestock, chicken, shrimp and fish populations. Infectious diseases can be very damaging to such populations and treatments are very costly.

WO 03/035176 describes a device which is particularly effective in stimulation of the immune system of humans and animals. This device is adapted to the application of time dependent electromagnetic fields to a part of the body of a living organism. The applied signal has a spectrum of frequencies in which some frequencies or frequency areas are more strongly present than others. Such a device can support a therapy in which afflictions involving inflammation and infection can be treated.

The system for electromagnetic stimulation described in WO 03/035176 is a small-scale system only suitable for the treatment of a single organism. Moreover, no indications regarding the shape of the signal are given. Effective electromagnetic stimulation of large populations on a large-scale and the particular type of signals used therein are not addressed in the prior art. For instance, livestock populations are usually kept in large-area buildings or other spaces of large dimensions. Usually stables and sheds for cows, chickens and pigs or ponds for breeding fish have typical dimensions of at least tens to hundreds of meters. Without special measures, controlled electromagnetic stimulation of such large areas is difficult and would require large amounts of energy. Moreover, when using the device in more remote areas a battery fed system with a solar and/or wind energy supply is necessary. In that case reduction in power consumption is a very important aspect.

Moreover, the buildings where livestock is kept vary in size and construction. The transducers installed in such buildings are tailor made. Consequently, the impedance of these electromagnetic transducers will - to a certain extent - vary from building to building. These variances and deviations in the load of the driving electronics of the electromagnetic transducers will affect the electromagnetic signal produced. This will negatively influence the effectiveness of the stimulation treatment.

Object of the invention

It is an object of the invention to reduce or eliminate at least one of the drawbacks of the prior art and to provide a device which is adapted for producing an electromagnetic field signal which is suitable for effective stimulation of a process, in particular the immune response.

## Summary of the invention

The invention relates to the observation that signals of specific shape are required to achieve an effective and beneficial stimulation of the processes within a living organism. It involves the recognition that a electromagnetic signal comprising one or, preferably, a superposition of at least two periodic electromagnetic signals of a particular shape is especially effective in the stimulation of biological processes, including stimulation of the immune system. As this electromagnetic signal causes an effective stimulation of the processes, including the immune response, in living organisms, small amplitude signals can be used thereby reducing the amount of energy needed to generate these fields. Moreover, the signal according to the invention can be generated by using relatively simple and cost effective electric components.

One aspect of the invention relates to a device for applying an electromagnetic field adapted to stimulate processes, such as the immune response, within living organisms when coupled to at least part of a body such organism. The device comprises a driving means, such as a digital signal generator, for generating a time varying drive signal and one or more transducers, such as specially adapted electromagnetic coil structures, which are responsive to the drive signal of the signal generator. Preferably the transducer is suitable for generating electromagnetic fields over large areas.

The transducer generates a time varying signal B(t) comprising the electromagnetic field which is very effective for stimulating processes within the body. Signal B(t) comprises one or, preferably, a superposition of at least two periodic base functions $b_i(t)$ (i=1,2,3,...), wherein the functions $b_i(t)$ are defined as:

$$b_i(t) = a_i * (2\exp(-\omega_i t) - (1+\exp(-\omega_i T_i/2)))$$

for $0 \leq t \leq T_i/2$

$$b_i(t) = -b_i(t-T_i/2)$$

for $T_i/2 \leq t \leq T_i$

$T_i$ is the period of the function $b_i(t)$, $a_i$ is an amplitude of the function and $\omega_i$ is a characteristic frequency which determines to a large extent the shape of the signal $b_i(t)$. It has been experimentally determined that such electromagnetic field provides an effective stimulation of a process within the body of a living organism.

Typically, the amplitude $a_i$ (i=1,2,3,...) is chosen such that the peak amplitude of B(t) at the treatment positions will be in the range between 1 nT to 1 mT, preferably within the range between 0.01 $\mu$T to 10 $\mu$T. Due to the effective shape of signal B(t) for electromagnetic stimulation, even small amplitudes signals will be sufficient to generate advantageous stimulation. The use of this signal thus drastically reduces energy consumption in large area and large scale applications.

In one embodiment each of said characteristic frequencies $\omega_i$ (i=1,2,3,...) is chosen to substantially match a desired characteristic frequency $\omega_o$. This way all base functions $b_i(t)$ have the same characteristic frequency $\omega_o$. Typically, the characteristic frequencies $\omega_i$ or the common characteristic frequency $\omega_o$ are chosen from a range between 200 and 20,000 rad.s$^{-1}$, more preferably between 500 and 15,000 rad.s$^{-1}$, in particular between 1000 and 10,000 rad.s$^{-1}$.

In one aspect of the invention the characteristic frequency $\omega_1$ of the transducer, which is determined by the R/L ratio, is chosen to substantially match the desired characteristic frequency $\omega_o$ of the signal. Here, R represents the resistance and L the inductances of the inductive coil(s) in said transducer. If the transducer is driven by a block-wave type drive signal an electromagnetic signal B(t) is generated which has optimal stimulation effects. Particular transducer structures are described in more detail in a related application with title "Coil structure for electromagnetic stimulation of processes within a living organism, device using such coil structure and method of driving", which is hereby incorporated by reference in this application.

In an embodiment of the invention at least one of the periods $T_i$ (i =1,2,3...) is chosen from a range between 0,01 ms and 1000 ms, preferably between 0,1 ms and 100 ms. Typically periods $T_i$ (i=1,2,3...) have different values. Preferably, at least one of said periods $T_i$ substantially matches one of a first group of periods $T_i'=1/f_i$ or a second group of periods $T_i''=(B_{loc}/B_o) \cdot (1/f_i)$ wherein $f_1$=10 Hz, $f_2$=700 Hz, $f_3$=750 Hz, $f_4$=2200 Hz. $B_{loc}$ is the local earth magnetic field at the position of the device and $B_o$=47 $\mu$T. Here, the scaling behavior of the frequency with the $B_{loc}/B_o$-ratio was experimentally determined. Scaling behavior with the ambient magnetic field was also observed in US 5,290,409.

Selection of the periods $T_i$ (i =1,2,3...) according to one or a combination of the above mentioned selection criteria will provide an electromagnetic field signal which is particularly effective and advantageous for use in electromagnetic stimulation in large-scale and large-area applications.

In an embodiment of the invention the device comprises a signal generator which supplies a signal to an amplifier for driving the electromagnetic transducer. Commonly known lineair amplifiers are not suitable for driving large area transducers. Such amplifier would consume too much energy. In one embodiment of the invention the amplifier is a switching amplifier, preferably a pulse width modulation amplifier or a class D amplifier. Such amplifiers have a high power conversion efficiency and reduced power dissipation. As a result less cooling is needed thereby allowing compact and simple circuitry.

In yet a further aspect of the invention the driving means is adapted to generate block-wave signals, preferably adapted to produce a driving voltage signal V(t) comprising one block-wave signal or, preferably, a superposition of at least two block-wave signals $v_i(t)$ (i=1, 2, 3,...) wherein each of the block-wave signals $v_i(t)$ has a corresponding period $T_i$. Block-wave signals can be easily generated by a digital signal generator and make optimal use of the voltage power supply in the device. In one embodiment the device is battery fed.

In a further embodiment the device comprises a signal compensation means for compensating the drive signal for variations in the characteristic frequency $\omega_1$ = R/L of the transducer. Such variations originate from variations in the impedance of the transducer.

Preferably the compensation means is arranged between the signal generator and the amplifying means. The compensation means comprises an active circuit with a characteristic frequency which substantially matches the desired characteristic frequency $\omega_o$ of the signal.

In one embodiment the compensation means comprises an RC circuit wherein the resistor $R_o$ and the capacitor $C_o$ of the RC circuit is chosen such that the $R_oC_o$ product substantially matches the desired characteristic frequency $\omega_o$ of the signal. The use of the RC circuit thus allows very simple and cost effective load adjustments and eliminates and/or reduces the detrimental effects of variations in the impedance of the transducer on the desired shape of the electromagnetic field signal.

The compensation means can also comprise an inductive active circuit or a combined capacitive/inductive active circuit having at least one characteristic frequency which matches the desired characteristic frequency $\omega_o$ of the signal. The compensation means thus allows the device to generate an electromagnetic signal of a preferred shape regardless of variations in the impedance of the transducer.

The invention further relates to a device for electromagnetic field stimulation of a process within a living organism when applied to at least part of a body, which comprises a driving means for generating a time varying drive signal, at least one transducer responsive to said drive signal for generating a time varying electromagnetic field and wherein said electromagnetic field contains a superposition of at least two periodic functions, each of said functions having a characteristic frequency $\omega_o$ determining the shape of said functions. The device further comprises a pulse width modulation amplifier and/or signal compensation means for compensating said drive signal for deviations in the characteristic frequency of said transducer $\omega_1$=R/L from said characteristic frequency $\omega_o$ arranged between said driving means and said transducer. The use of a pulse width modulation amplifier and/or the signal compensation means in the device according to the invention provides very effective driving electronics for large area and large scale electromagnetic transducers for stimulation of a process within a living organism.

Brief description of the drawings

The invention will be further explained by means of the description of exemplary embodiments, reference being made to the following figures:

Fig. 1 represents a schematic drawing of a device according to an embodiment of the invention.
Fig. 2 represents a schematic drawing of the shape of a preferred periodic base signal $b_i(t)$.
Fig. 3 illustrates the results of experiments on phagocyte cells treated with an electromagnetic field signal.
Fig. 4 illustrates the results of in vivo experiments on infected fantail goldfish treated with an electromagnetic field signal.
Fig. 5 illustrates the results of in vivo experiments on infected chicken broilers treated with an electromagnetic field signal.
Fig. 6 represents a graph regarding the improved feed conversion of chicken broilers treated with an electromagnetic field signal.
Fig. 7 represents a schematic drawing of the driving electrons of a driving means according to an embodiment of the invention.

Description of exemplary embodiments

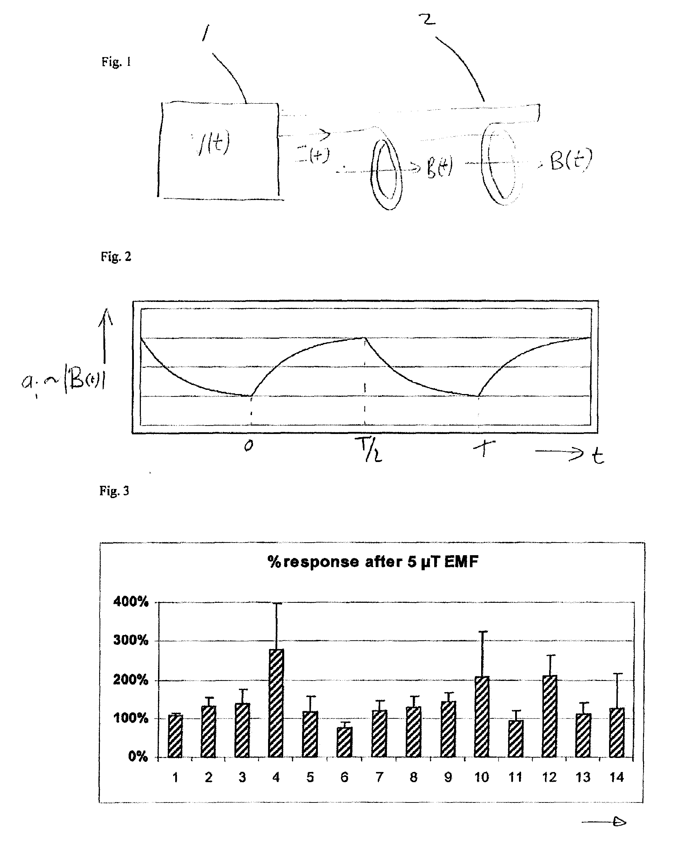

Fig. 1 shows a schematic representation of a device for electromagnetic stimulation according to the present invention. The device comprises a driving means 1 for generating a voltage signal V(t) which drives the electromagnetic transducer 2. The transducer 2 comprises one or more electromagnetic coils having together a certain inductance L and resistance R. In response to the driving signal V(t), a current I(t) runs through transducer, generating a electromagnetic field B(t). Typically, in large area and large scale applications the electromagnetic coils form distributed coil structures. These distributed structures are arranged over or under a surface area S on which the living organisms are kept. The specific transducer structures are described in more detail in a related application with title "Coil structure for electromagnetic stimulation of processes within a living organism, device using such coil structure and method of driving".

The driving means generates a driving signal which is fed to the electromagnetic transducer. In response, the transducer generates a time varying signal B(t) comprising an electromagnetic field which is very effective in stimulating processes within the body. The low frequency electromagnetic signal B(t) comprises a single base signal or, preferably, a composite signal. The composite signal contains a superposition of at least two periodic base signals $b_i(t)$ (i=1,2,3,...) wherein each of these base signals has a shape as illustrated in Fig. 2. The periodic base signal $b_i(t)$ is defined as:

$$b_i(t) = a_i * (2 \exp(-\omega_i t) - (1 + \exp(-\omega_i T_i/2)))$$

for $0 \le t \le T_i/2$

$$b_i(t) = -b_i(t - T_i/2)$$

for $T_i/2 \le t \le T_i$

Here $T_i$ is the period of the signal $b_i(t)$, $a_i$ is an amplitude of the signal and $\omega_i$ is a characteristic frequency of the signal. The characteristic frequency $\omega_i$ determines the rise and fall time of the signal and thus determines to a large extent the shape of the signal.

The graphs in Figures 3 to 6 show results from in vitro and in vivo experiments in which effects on the immune response were explored to various pathogens of exposure using the composite low frequency electromagnetic signal of the present invention. The signal comprised shaped waveforms $b_i(t)$ as described in relation to the base signals of Fig. 2. Typically, base frequencies $f_i = 1/T_i$ between 250 and 5000 Hz were used. The experiments described in the Figures 3 to 6 relate to a daily, 30 minutes electromagnetic stimulation treatment using a signal composed of the base frequencies 700 and 750 Hz. The functions $b_i(t)$ were chosen to have the same characteristic frequency $\omega_o$ of around 1900 rad.s$^{-1}$. Various electromagnetic field strengths between 100 nT and 50 $\mu$T were used.

Fig. 3 shows the results of a series of in vitro experiments on phagocytes. The figure depicts the Oxygen burst in phagocyting cells relative to the control wherein each run represents 48 samples (total confidence level p<0.0001). Reactive oxygen species (ROS) production in electromagnetically stimulated common carp head kidney-derived phagocytes was determined as a measure for immune activation. The measurements were based on the reduction of the salt nitro blue tetrazolium (NBT) by oxygen. Such reduction results in a blue coloration and can be measured using spectrophotometrics. From the experimental results it followed that exposure to an electromagnetic field of 5 $\mu$T and 1,5 mT led to 42% and 33% increase in immune activity, respectively, compared to negative control values.

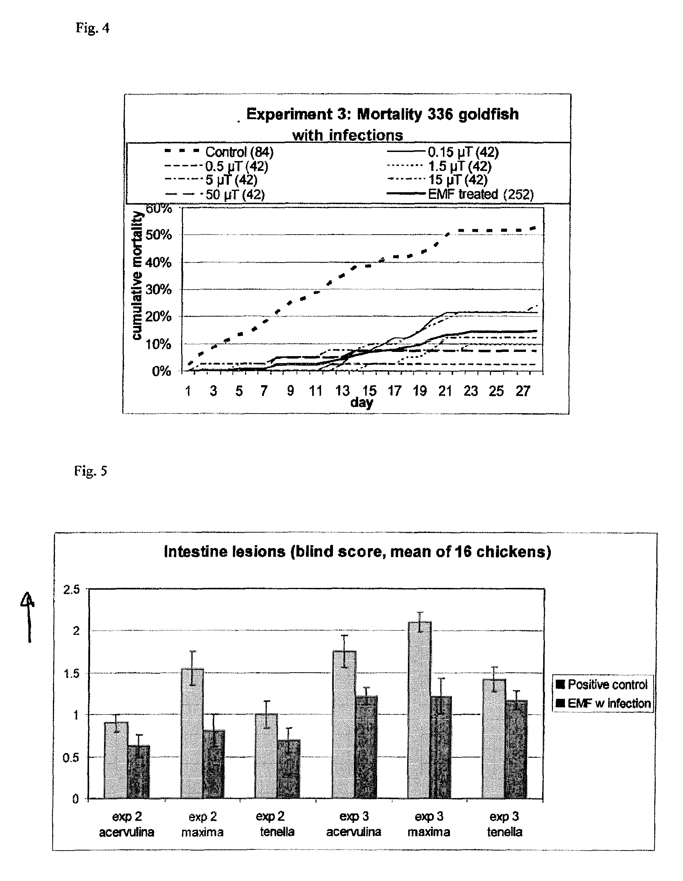

Fig. 4 shows results in vivo experiments on fantail goldfish (Carrassius Auratus spp.). Electromagnetic stimulation experiments were performed using six different field strengths ranging from 0.15 $\mu$T to 50 $\mu$T. The goldfish were heavily infected with Ecto parasites (Gill parasites) such as Dactylogyrus/Gyrodactylus, Trichodina, Chilodinella and Costia. These types of parasite infections occur frequently at the breeding stage of the fish and increase in intensity during storage and international transport due to the fact that large populations are packaged in one volume. Such infections and subsequent secondary bacterial infections cause high mortality if not treated.

The results in Fig. 4 show that the control group suffered a mortality rate up to 52% on day 28. In contrast, the average mortality rate of the electromagnetically treated fish was 15% at day 28. The effectiveness of the treatment reduces when using fields smaller than 0,05 $\mu$T. These results were reproducible and show that the low energy electromagnetic treatment using the composite electromagnetic signals generated by the device of the present invention results in a decrease in mortality at all field strength levels used.

Fig. 5 illustrates a series of in vivo experiments on 560 commercial broiler chickens, which were exposed to infection pressure from Coccidiosis. The graphs show that Coccidial lesion of intestines due to Eimeria Acervulina and Eimeria Maxima were significantly lower in group treated with an electromagnetic field. Treatment with a 6.5 $\mu$T composite electromagnetic field signal reduced intestinal lesions up to 40%.

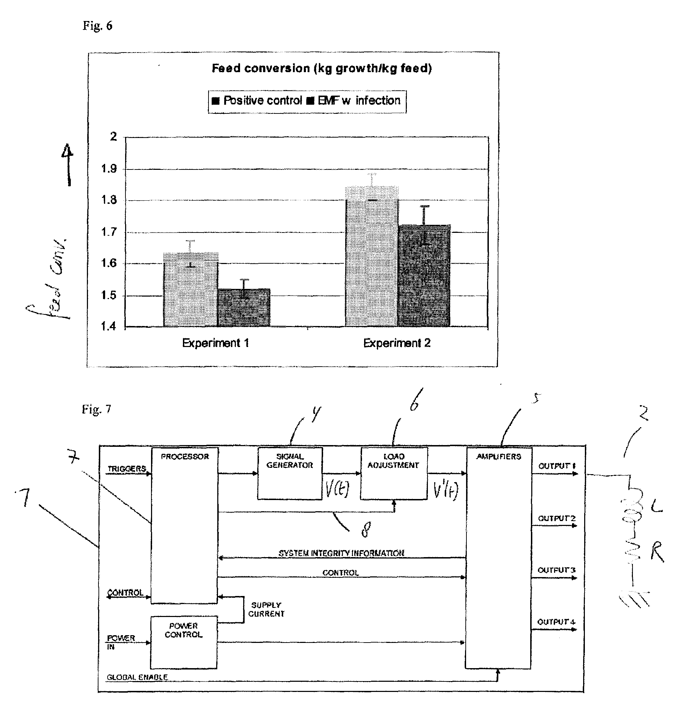

Fig. 6 depicts the feed conversion (i.e. the ratio between the growth of the chickens in kilograms and the feed in kilograms) of treated and non-treated chickens in the experiments as described in relation to Fig. 5. A significant and economically relevant improvement in feed conversion up to 8% is achieved by electromagnetic treatment of chickens with Coccidiosis infection. The improvement indicate that the electromagnetic stimulate the health and thus the growth per unit of feed of the chickens.

Further experiments show that particular effective stimulation can be achieved by selecting base frequencies from a first group of frequencies $f_1$=10 Hz, $f_2$=700 Hz, $f_3$=750 Hz, $f_4$=2200 Hz and/or a second group of frequencies equal to the frequencies of the first group multiplied with a factor $B_{loc}/B_o$, wherein $B_{loc}$ is the local earth magnetic field at the position of the device and $B_o$=47 $\mu$T.

The electromagnetic signal is generated by a driving means 1 comprising driving electronics as schematically illustrated in Fig. 7. A signal generator 4 provides a driving signal V(t) to the input of one or more amplifiers 5. The signal generator 4 is typically a digital signal generator, which is capable of generating a driving signal V(t) comprising one block-wave signal or, preferably, a superposition of at least two block-wave signals $v_i(t)$(i=1,2,3,...), wherein each of the block-wave signals $v_i(t)$ has a corresponding period $T_i$. Preferably, the base functions $b_i(t)$ have the same characteristic frequency $\omega_o$. In that case the desired shape of the signal is determined by choosing the characteristic frequency $\omega_1$=R/L of the inductive coil(s) in the transducer 2 to match approximately the desired characteristic frequency $\omega_o$.

The driving means further comprises a compensations means 6 which is arranged between the signal generator 4 and the amplifiers 5 as shown in Fig.7. The compensation means 6 is able to compensate for deviations $\Delta\omega$ between the desired characteristic frequency $\omega_o$ and the characteristic frequency $\omega_1$=R/L of the inductive coil(s). These deviations $\Delta_\omega$ are caused by various reasons such as geometrical variations in impedance of the coils or (geometrical) restrains in matching $\omega_1$ to the desired characteristic frequency $\omega_o$.

In order to generate the desired electromagnetic field B(t), a current I(t) should be generated in the coil(s) of the transducer 2. This is done by applying a voltage signal V(t) comprising one or, preferably, a superposition of at least two block-wave signals $v_i(t)$(i=1,2,3,...) to the input of one or more amplifiers which drive the transducer. Here the characteristic frequency of the transducer $\omega_i$=R/L approximately matches the characteristic frequency $\omega_o$ of the desired signal. If however $\omega_1$ deviates with a value $\Delta\omega$ from $\omega_o$ then an adjusted voltage V'(t)=V(t)-L$\Delta\omega$I(t) should be generated in order to obtain the desired electromagnetic field signal B(t). V'(t) could be generated digitally, however this solution requires expensive signal processing hardware.

In one aspect of the invention a compensation means 6 allows the generation of the adjusted voltage V'(t) with simple low power, analog components so that deviations in the impedance of the transducer are compensated. In the compensation means 6 the voltage V(t) of the signal generator is applied to an RC circuit having a resistor $R_o$ and a capacitor $C_o$ such that the $R_oC_o$ product substantially matches the desired characteristic frequency $\omega_o$ of the signal. Here a relatively high resistance $R_o$ can be chosen such that the energy dissipation in the circuit can be kept low. By using simple analog addition and subtraction circuitry, which is well known in the art, the adjusted voltage V'(t) can be constructed in a simple way even when V(t) is a more complex signal constructed by the addition of various block-wave functions $v_i(t)$.

The use of the RC circuit thus allows very simple and cost effective load adjustments and eliminates and/or reduces the detrimental effects of variations in the impedance of the transducer on the desired shape of the electromagnetic field signal. The compensation means can also comprise inductive active circuitry or combined capacitive/inductive active circuitry having at least one characteristic frequency which substantially matches the desired characteristic frequency $\omega_o$ of the signal.

The voltage V(t) of the signal generator or, when applicable, the compensated voltage signal V'(t) is preferably offered to the input of a pulse width modulation amplifier or a class D amplifier, which have a high power conversion efficiency and reduced power dissipation compared to a conventional linear amplifier. As a result less cooling is needed when thereby allowing compact and simple circuitry. The energy considerations in the design of the driving means are especially important when the driving means is battery fed, which is required when the stimulation treatment is used in more remote areas.

The driving means in Fig. 7 can further comprise a processor 7 for control and automation of the signal generation processes. For instance the driving means can include further circuitry which is able to determine the characteristic frequency $\omega_1$ of the transducer. Using this frequency the processor can instruct the compensation means via a line 8 to generate a compensated voltage signal V'(t).

The invention is not limited to the embodiments described above, which may be varied within the scope of the accompanying claims.

**Claims**

1. Device for applying an electromagnetic field for stimulation of a process within a living organism when applied to at least part of a body, comprising:

   - driving means for generating a time varying drive signal,
   - at least one transducer responsive to said drive signal for generating a time varying signal B(t) comprising said electromagnetic field, and

wherein said signal B(t) comprises one periodic signal $b_i(t)$ or a superposition of two or more periodic signals $b_i(t)$ (i=1,2,3,...), each signal $b_i(t)$ being defined as:

$$b_i(t) = a_i * (2\exp(-\omega_i t) - (1 + \exp(-\omega_i T_i/2)))$$

for $0 \leq t \leq T_i/2$

$$b_i(t) = -b_i(t - T_i/2)$$

for $T_i/2 \leq t \leq T_i$

wherein $T_i$ is the period of $b_i(t)$, $a_i$ is an amplitude of $b_i(t)$ and $w_i$ is a characteristic frequency determining the shape of the signal $b_i(t)$.

2. Device according to claim 1, wherein said characteristic frequencies $w_i$ (i=1,2,3,...) are chosen from a range between 200 and 20,000 $rad.s^{-1}$, more preferably between 500 and 15,000 $rad.s^{-1}$, in particular between 1000 and 10,000 $rad.s^{-1}$.

3. Device according to claims 1 or 2, wherein at least one of said periods $T_i$ (i =1,2,3...) is chosen from a range between 0,01 ms and 1000 ms, preferably between 0,1 ms and 100 ms.

4. Device according to any of claims 1 to 3, wherein at least two of said periods $T_i$ (i=1,2,3...) are chosen to have different values.

5. Device according to any of claims 1 to 4, wherein at least one of said periods $T_i$ substantially matches one of the periods defined by a first group of periods $T_i'=1/f_i$ or a second group of periods $T_i''= (B_{loc}/B_o) \cdot (1/f_i)$ wherein $f_1=10$ Hz, $f_2=700$ Hz, $f_3=750$ Hz, $f_4=2200$ Hz, $B_{loc}$ is the local earth magnetic field at the position of the device and $B_o=47$ $\mu$T.

6. Device according to any of claims 1 to 5, wherein said device further comprises amplifying means, preferably a switching amplifier or a pulse width modulation amplifier, arranged between said driving means and said transducer.

7. Device according to any of claims 1 to 6, wherein said driving means comprises a signal generator, preferably a digital voltage generator, which is adapted to generate a driving signal V(t) comprising one block-wave signal or a superposition of at least two block-wave signals $v_i$ (t) (i=1,2,3,...), wherein each of said block-wave signals $v_i(t)$ has a corresponding period $T_i$.

8. Device according to claims 1-7, wherein each of said characteristic frequencies $\omega_i$ (i=1,2,3,...) substantially matches the characteristic frequency of said inductive coil $\omega_1=R/L$.

9. Device according to any of claims 1-7, wherein each of said characteristic frequencies $\omega_i$ (i=1,2,3,...) substantially matches a characteristic frequency $\omega_o$ and wherein said device further comprises signal compensation means for compensating said drive signal for deviations in the characteristic frequency of said transducer $\omega_1=R/L$ from said characteristic frequency $\omega_o$.

10. Device according to claim 9, wherein said signal compensation means is arranged between said driving means and said amplifying means.

11. Device according to claim 9 or 10, wherein said signal compensation means comprises an RC circuit wherein resistor $R_o$ and capacitor $C_o$ of said RC circuit is chosen such that the product $R_o \cdot C_o$ substantially matches said characteristic frequency $\omega_o$.

12. Device for electromagnetic field stimulation of a process within a living organism when applied to at least part of a body, comprising:

- driving means for generating a time varying drive signal,
- at least one transducer responsive to said drive signal for generating a time varying electromagnetic field and wherein said electromagnetic field contains a superposition of at least two periodic functions, each of said functions having a characteristic frequency $\omega_o$ determining the shape of said functions,

wherein said device further comprises a pulse width modulation amplifier and/or signal compensation means for compensating said drive signal for deviations in the characteristic frequency of said transducer $\omega_1 = R/L$ from said characteristic frequency $\omega_o$ arranged between said driving means and said transducer.

**13.** Method for applying an electromagnetic field for stimulation of a process within a living organism when applied to at least part of a body , comprising the steps of:

- generating a time varying drive signal,
- using at least one transducer responsive to said drive signal for generating a time varying signal B(t) comprising said electromagnetic field, and

wherein said signal B(t) comprises one periodic signal $b_i(t)$ or a superposition of at least two periodic signals $b_i(t)$ (i=1,2,3,...), each signal $b_i(t)$ being defined as:

$$b_i(t) = a_i * (2\exp(-\omega_i t) - (1+\exp(-\omega_i T_i/2)))$$

for $0 \le t \le T_i/2$

$$b_i(t) = -b_i(t-T_i/2)$$

for $T_i/2 \le t \le T_i$
wherein $T_i$ is the period of $b_i(t)$, $a_i$ is an amplitude of $b_i(t)$ and $\omega_i$ is a characteristic frequency determining the shape of the signal $b_i(t)$.

**14.** Method of driving a transducer in a device according any of claims 1 to 11, wherein said transducer is driven by a driving signal V(t) containing one block-wave signal or a superposition of two or more block-wave signals $v_i(t)$ (i=1, 2, 3,...), wherein each of said block-wave signals $v_i(t)$ has a corresponding period $T_i$.

Fig. 1

Fig. 2

Fig. 3

Fig. 4

**Experiment 3: Mortality 336 goldfish with infections**

Legend:
- ▪ ▪ ▪ Control (84)
- ────── 0.15 µT (42)
- ─ ─ ─ ─ 0.5 µT (42)
- ·········· 1.5 µT (42)
- ─ · · ─ 5 µT (42)
- ─ ·· ─ ·· 15 µT (42)
- ─ ─ · 50 µT (42)
- ────── EMF treated (252)

Fig. 5

**Intestine lesions (blind score, mean of 16 chickens)**

Legend:
- ▪ Positive control
- ▪ EMF w infection

Categories: exp 2 acervulina, exp 2 maxima, exp 2 tenella, exp 3 acervulina, exp 3 maxima, exp 3 tenella

Fig. 6

**Feed conversion (kg growth/kg feed)**

■ Positive control ■ EMF w infection

Fig. 7

EP 2 020 250 A1

European Patent Office

**PARTIAL EUROPEAN SEARCH REPORT**

Application Number

which under Rule 45 of the European Patent Convention shall be considered, for the purposes of subsequent proceedings, as the European search report

EP 07 11 3241

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 561 968 B1 (DISSING STEEN [DK] ET AL) 13 May 2003 (2003-05-13) * abstract; figures 1A-B,2,7,8 * * column 4, lines 6-65 * * column 6, line 63 - column 7, line 25 * * column 11, line 31 - column 13, line 10; claims 1,11 * ----- | 1-12,14 | INV. A61N2/00 |
| X | EP 0 601 545 A (ELECTRO BIOLOGY INC [US]) 15 June 1994 (1994-06-15) * abstract; figures 1,2 * * page 4, line 51 - page 5, line 17 * * page 5, line 49 - page 6, line 38 * ----- | 1-12,14 | |
| X | US 5 951 459 A (BLACKWELL LYMAN L [US]) 14 September 1999 (1999-09-14) * abstract; figures 1B,4A-C,5 * * column 3, line 48 - column 4, line 53 * * column 6, line 48 - column 7, line 42 * ----- | 1-12,14 | |
| X | US 7 160 241 B1 (HERBST EWA [US]) 9 January 2007 (2007-01-09) * abstract; figures 1,5-7 * * column 4, line 34 - column 6, line 44 * ----- | 1-12,14 | TECHNICAL FIELDS SEARCHED (IPC) A61N |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 12 September 2007 | Lahorte, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

12

European Patent
Office

INCOMPLETE SEARCH
SHEET C

Application Number

EP 07 11 3241

Claim(s) not searched:
        13

Reason for the limitation of the search (non-patentable invention(s)):

Article 52 (4) EPC - Method for treatment of the human or animal body by therapy

## ANNEX TO THE EUROPEAN SEARCH REPORT
## ON EUROPEAN PATENT APPLICATION NO.

EP 07 11 3241

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2007

| Patent document cited in search report | | Publication date | Patent family member(s) | | Publication date |
|---|---|---|---|---|---|
| US 6561968 | B1 | 13-05-2003 | NONE | | |
| EP 0601545 | A | 15-06-1994 | JP | 6233825 A | 23-08-1994 |
| | | | US | 5338286 A | 16-08-1994 |
| US 5951459 | A | 14-09-1999 | US | 5997464 A | 07-12-1999 |
| | | | US | 6186941 B1 | 13-02-2001 |
| US 7160241 | B1 | 09-01-2007 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3890953 A
- US 5183456 A
- US 5290409 A
- WO 03035176 A

### Non-patent literature cited in the description

- **SIMKÓ et al.** *Eur. J. Cell Biol.,* 2001, vol. 80, 562-566
- **LUPKE et al.** *Free Radic. Res,* 2004, vol. 38, 985-993
- **BLANK et al.** *Bio-electrochem. and Bioenerg.,* 1992, vol. 33, 109-114
- *Mol. Biol. Cell,* 1995, vol. 6, 466a
- **GOODMAN et al.** *Bioelectro-chem. and Bioenerg.,* vol. 33, 115
- **SIMKÓ et al.** *J. Cell. Biochem.,* 2004, vol. 93, 83-92
- **MONSELISE et al.** *Biochem. & Biophys. Res. Com.,* 2004, vol. 302 (2), 427-434
- **DE BRUYN et al.** *Environ. Res,* 1994, vol. 65 (1), 149-160
- **MARKOV et al.** Bioelectromagnetics. Springer, 2006, 213-225